(19) Europäisches Patentamt
European Patent Office
Office européen des brevets

(11) Publication number: **0 280 394 B1**

# (12) EUROPEAN PATENT SPECIFICATION

(45) Date of publication of patent specification: **14.12.94**

(51) Int. Cl.$^5$: **A61K 9/50**, A61K 31/53

(21) Application number: **88300529.0**

(22) Date of filing: **22.01.88**

The file contains technical information submitted after the application was filed and not included in this specification

## (54) Phospholipid delivery vehicle for aqueous-insoluble active ingredients.

(30) Priority: **27.01.87 US 7338**

(43) Date of publication of application:
**31.08.88 Bulletin 88/35**

(45) Publication of the grant of the patent:
**14.12.94 Bulletin 94/50**

(84) Designated Contracting States:
**AT BE CH DE ES FR GB GR IT LI LU NL SE**

(56) References cited:
**EP-A- 0 118 316**
**FR-A- 2 455 458**
**GB-A- 2 018 712**
**US-A- 4 610 868**

**CHEMICAL ABSTRACTS, vol. 103, no. 18, 4th November 1985, page 329, abstract no.147032r, Columbus, Ohio, US; A.D. WICKES et al.: "Pharmacokinetics of hexamethylmelamine administered via the i.p. route in an oil emulsion vehicle",& CANCER TREAT. REP. 1985, 69(6), 657-62**

(73) Proprietor: **VESTAR, INC.**
**650 Cliffside Drive**
**San Dimas California 91773 (US)**

(72) Inventor: **Forssen, Eric Anton**
**5181 Princess Ann Road**
**La Canada California 91011 (US)**

(74) Representative: **Goldin, Douglas Michael et al**
**J.A. KEMP & CO.**
**14, South Square**
**Gray's Inn**
**London WC1R 5LX (GB)**

**EP 0 280 394 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF INVENTION

This invention relates to phospholipid-encapsulated medicinal agents. It is directed in one aspect to phospholipid-encapsulated hexamethylmelamine. In another aspect it relates to the use of such compositions to deliver medicinal agents to the body, as for example to tumor cells.

BACKGROUND

Although a significant number of substances are known to have antitumor activity, problems have persisted in many cases in developing compositions and methods for safely and effectively delivering such substances to tumor cells. The general toxicity of many anticancer agents prevents their being administered in free form in the body. Many anticancer agents are not sufficiently soluble or stable in the aqueous environment to allow injection or other effective administration. Furthermore, it is frequently useful to control the size of delivery agents in order to achieve targeting to tumor cells or to allow filtration for the purpose of removing deleterious components such as bacteria. It is also important to achieve a composition which, apart from being non-toxic, is biocompatible.

Phospholipid-encapsulated delivery vehicles have been used to overcome such problems in certain cases. It is known, for example, that some aqueous-insoluble drugs can be incorporated into the lipophilic region within the phospholipid bilayer of a liposome to achieve an aqueous-soluble, relatively non-toxic and biocompatible delivery vehicle. Not all aqueous-insoluble materials are susceptible to such a composition, however.

GB-A-2018712 describes xenobiotic delivery vehicles which are microreservoirs comprising an inner core of a drug such as adriamycin or imidocarb, and a cholesterol ester, such as cholesterol oleate, or triglyceride, such as glycerol trioleate, and one or more outer layers of phospholipid material.

US-A-4610868 describes lipid matrix carriers of 500 to 100 000 nm in diameter, which are produced by forming an emulsion of water, a pharmaceutical, and hydrophobic and amphiphatic lipid components, and extruding this emulsion through an orifice into an organic solvent such as acetone.

Hexamethylmelamine (HXM) is an example of an anticancer agent which has received only limited use due to its poor aqueous solubility. Oral administration of HXM yields variable absorption and erratic drug concentrations in the plasma. Ames et al., Cancer Treatment Reports, Vol. 66, No. 7, pp. 1579-1581 (July 1982). Gentisate and hydrochloride salts of HXM have resulted in severe local irritation upon intravenous administration to humans. Recent attempts to formulate HXM in an intravenously-acceptable preparation have focused on incorporating the drug into fat emulsions, and have achieved HXM concentrations of 2 mg/ml or more. Intraperitoneal formulations have also focused on fat emulsions such as that formed with the oil emulsion vehicle Intralipid (Cutter Laboratories, Berkeley, California), discussed by Wickes et al., in Cancer Treatment Reports, Volume 69, No. 6, pp. 657-662 (June 1985). Although such formulations succeed in increasing the concentration of HXM to levels suitable for affecting tumor cells, they do not address the problem of targeting tumor cells specifically through use of phospholipid-encapsulated vesicles of an appropriate size. Nor do they address the problem of sterilization where the medicinal or other component may not be heat-stable since such a preparation can not be sterile filtered.

Accordingly, it is an object of the present invention to provide new compositions for the formulation and delivery of aqueous-insoluble medicinal agents to the body. In one aspect, the invention provides compositions for the formulation and delivery of anticancer agents, including hexamethylmelamine.

It is another object of the present invention to provide methods for manufacturing, sterilization and use of such compositions to deliver medicinal agents to the body, and in particular to tumor cells.

The present invention involves compositions suitable for the delivery of a substantially aqueous-insoluble active ingredient to humans or animals comprising vesicles in a pharmaceutically acceptable carrier, wherein said vesicles are of a size of 30 to 200 nanometers in diameter and comprise (1) a substantially aqueous-insoluble active ingredient in mixture with trilauroylglycerol and (2) an encapsulating layer including a phospholipid material. It is thought that the emulsified vesicles have a roughly spherical outer monolayer of phospholipids with hydrophobic tails of the phospholipid molecules oriented inwardly toward the medicinal active ingredient/lipid triglyceride phase.

A preferred active ingredient is the anticancer agent hexamethylmelamine. The phospholipid outer coating preferably comprises one or more phospholipid materials having from 12 to 20 carbons in the alkyl chains. Distearoylphosphatidylcholine and distearoylphosphatidylglycerol are preferred in the case of the active ingredient hexamethylmelamine. Cholesterol may also be added to the compositions. Preparation of

the compositions may be carried out using standard procedures in an appropriate saline or saccharide-based carrier solution. Glycerol may also be added to the aqueous carrier to minimize aggregation of the final compositions.

## BRIEF DESCRIPTION OF THE DRAWING

Figure 1 is a cross-sectional schematic illustration of the theoretical structure of the delivery vehicle of the present invention.

## DETAILED DESCRIPTION OF THE INVENTION

As indicated above, the present invention involves the encapsulation and improved delivery of aqueous-insoluble active ingredients, and in particular insoluble anticancer agents such as hexamethylmelamine, in phospholipid vesicles. The present compositions may be used in some cases where other delivery vehicles, such as liposomes, are not satisfactory.

Hexamethylmelamine (HXM), or 2,4,6-Tris(dimethylamino)-s-triazine, is an anticancer compound that is very similar structurally to the alkylating agent triethylenemelamine. Its structure is as follows:

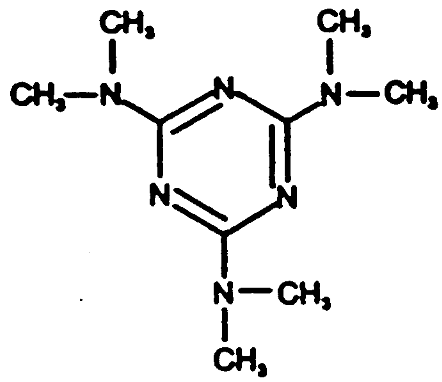

As discussed above, the poor aqueous solubility of HXM has hindered its usefulness in anticancer therapy. Investigations relating to the present invention have shown that improved solubilization of HXM in a phospholipid vesicle may be achieved with the present delivery vehicles as compared to liposomal compositions.

The present compositions are thought to have a structure as shown in Fig. 1 in cross-section. The delivery vehicle may be roughly spherical in shape. The inner phase of the delivery vehicle includes the active ingredient dissolved in the lipid trilauroylglycerol (trilaurin). Because this inner phase is essentially lipophilic, it will form a stable association with an encapsulating monolayer of phospholipids. The hydrophilic nature of the outer surface of the encapsulating layer allows aqueous and in vivo solubilization, and may achieve other advantages associated with liposomal structures (including biocompatibility, isolation of active ingredient toxicity and targeting of tumor cells).

It is necessary to utilize an appropriate lipid triglyceride in order to achieve a satisfactory delivery vehicle. A given active ingredient may be soluble in a number of triglycerides, or it may be made soluble by, for example, altering pH or ionic strength of the mixture or by complexing the active ingredient with a second lipid-soluble agent. Nevertheless, not all lipid triglycerides that can solubilize a given active ingredient will necessarily be compatible with a stable phospholipid emulsion. For example, fully-saturated long chain triglycerides such as tripalmitin and tristearin solubilize HXM upon heating but tend to form a hard waxy composition upon cooling that cannot be satisfactorily emulsified with the phospholipids tested. Conversely, the long unsaturated alkyl chains in the triglyceride triolein will allow an emulsion with phospholipids, but are susceptible to oxidation. Shorter-chain triglycerides such as triacetin and tributyrin poorly solubilize HXM and do not form stable emulsions in the compositions tested.

In the case of, for example, the active ingredient HXM, the triglyceride trilaurin is preferred. This triglyceride will solubilize the active ingredient and is able to form stable emulsions.

The active ingredient to be used herein will typically be one or more compounds which are insoluble in aqueous media or which require enhanced solubilization to achieve useful concentration. With respect to HXM, for example, solubility of the free drug in saline solution has been reported to be as low as 0.070

mg/ml (Wickes et al., Cancer Treatment Reports, Volume 69, No. 6, pp. 657-662 (June 1985)) and as high as 0.20 mg/ml in water (Ames et al., Cancer Treatment Reports, Volume 66, No. 7, pp. 1579-1581 (July 1982)). A desirable concentration would exceed 1.0 mg/ml measured with respect to HXM content.

Although the active ingredients useful in the present invention will typically be agents that are difficult to solubilize in aqueous media, this need not necessarily be the case. So long as the active ingredient can be made compatible with the triglyceride phase, and so long as it is desirable to encapsulate the ingredient in a phospholipid monolayer, the present invention may yield a useful delivery vehicle. As discussed above, modifications to the pH or ionic strength of the mixture, or modifications to the active ingredient such as complexation, may be employed to render the ingredient triglyceride-soluble. Similar modifications may be made to allow formation of a stable phospholipid vesicle.

The outer phospholipid coating may be composed of a range of phospholipids including neutral phospholipids such as phosphatidylcholines and phosphatidylethanolamines, as well as ionic phospholipids such as phosphatidylglycerols and phosphatidylserines. Preferred phospholipids are those having from 12 to 20 carbons in their alkyl side chains. Cholesterol may also be added as component of the outer layer and is preferred in many cases.

Distearoylphosphatidylcholine is a particularly preferred phospholipid with the active ingredient HXM and the inner phase triglyceride trilaurin. The anion distearoylphosphatidylglycerol may also be added to yield a successful composition. Cholesterol is a preferred component in the outer coating. The molar ratio of ingredients in such a HXM composition will preferably range as follows:

| | |
|---|---|
| HXM: | 1 |
| Distearoylphosphatidylcholine: | 2-1 |
| Cholesterol: | 1 |
| Distearoylphosphatidylglycerol: | 0-1 |
| Trilaurin: | 4 |

The formation of the emulsified delivery vehicles may be achieved in a saline solution, as for example a 0.9% solution of sodium choloride in water, or in a saccharide or disaccharide solution, such as 5% dextrose or 9% lactose in water. In addition, it is often preferred to add glycerol to the mixture in a concentration of about 100 mM in order to reduce or eliminate any adverse tendency toward aggregation of the vesicles. Formation of the vesicles may be achieved using standard sonication techniques. Such is the case with the HXM compositions described herein.

Non-solubilized material may be removed from the mixture by centrifugation. Further purification may include filtration, for example, through a 5-micrometer filter needle to ascertain syringeability, and through a 0.45 and/or 0.22-micrometer filter to remove, e.g., bacterial contaminants. The final delivery vehicle vesicles will preferably be smaller than 100 nm in diameter, and preferably in the range 40-75 nm in diameter.

The following examples demonstrate the preparation and characterization of one form of the delivery vehicles of the present invention. Example 2 is given to compare one of the present compositions with a liposome-type delivery vehicle tested for use with HXM.

## EXAMPLE 1

### Preparation of a Hexamethylmelamine-Trilaurin Delivery Vehicle Encapsulated With Phospholipid.

Hexamethylmelamine was supplied by the National Cancer Institute, compound NSC-13875, lot number H739646. Solubility tests were run for HXM in a number of pure trigylcerides and it was determined that HXM was highly soluble (more than 50 mg/ml) in tributyrin, trihexanoin, tricaprylin, trilaurin and tripalmitin (Sigma Chemical Co., St. Louis, MO). Emulsions of HXM were formed by heating a measured quantity of the triglyceride to a liquid state and adding with stirring measured quantities of HXM, phospholipid (Avanti Biochemicals, Birmingham, AL), cholesterol (Sigma) and, finally, the aqueous solution phase. The solution was then sonicated under an inert atmosphere using a probe type sonicator (Sonics and Materials, Model VCS-500, Danbury, CT). The sample was then centrifuged at 750 g for ten minutes and the amount of precipitate estimated. An alternate composition was sought if the total precipitation was greater than about 20% of the starting material. Preferably, the precipitate fraction would be less than 10%.

Following filtration of the emulsion of delivery vehicles through a 5-micrometer filter needle to verify syringeability, the samples were filtered through 0.45 and/or 0.22 micrometer microfilters. They were then analyzed for total HXM concentration and for evidence of any HXM decomposition using thin layer

chromatography on silica gel 60 plates (Merck), high pressure liquid chromatography and/or UV/visible spectroscopy using a Perkin-Elmer Lamda 3B spectrophotometer.

The results of such procedures are summarized in Table 1.

TABLE 1

| Composition of Mixture[1/] | Triglyceride | Aqueous Phase | % Precipitation[2/] | Final HXM Concentration[3/] |
|---|---|---|---|---|
| HXM:DSPC:CHOL:DSPG:TRI | | | | |
| 1 : 3 : 1 : 0 : 3 | Tributyrin | Lactose, 9%<br>Glycerol, 100 mM | 10% | 0.61,0.70(n=2) |
| 1 : 2 : 1 : 0 : 4 | Tripalmitin | Dextrose, 5% | 100% | - |
| 1 : 2 : 1 : 0 : 4 | Tripalmitin | Dextrose, 5%<br>Glycerol, 100 mM | 100% | - |
| 1 : 2 : 1 : 1 : 4 | Tripalmitin | Dextrose, 5% | 100% | - |
| 1 : 2 : 1 : 1 : 4 | Tripalmitin | Dextrose, 5%<br>Glycerol, 100 mM | 100% | - |
| 1 : 2 : 1 : 0 : 4 | Trilaurin | NaCl, 0.9% | 10% | - |
| 1 : 2 : 1 : 0 : 4 | Trilaurin | NaCl, 0.9%<br>Glycerol, 100 mM | 5% | 2.5 |
| 1 : 2 : 1 : 0 : 4 | Trilaurin | Dextrose, 5% | 10% | - |
| 1 : 2 : 1 : 0 : 4 | Trilaurin | Dextrose, 5%<br>Glycerol, 100 mM | 5% | 3.0 |
| 1 : 1 : 1 : 1 : 4 | Trilaurin | NaCl, 0.9% | 10% | - |
| 1 : 1 : 1 : 1 : 4 | Trilaurin | NaCl, 0.9%<br>Glycerol, 100 mM | <5% | 3.0,2.2(n=1) |
| 1 : 1 : 1 : 1 : 4 | Trilaurin | Dextrose, 5% | 10% | - |
| 1 : 1 : 1 : 1 : 4 | Trilaurin | Dextrose, 5%<br>Glycerol, 100 mM | <5% | 3.1,2.2(n=1) |

1/ Molar ratios. Abbreviations: HXM -- hexamethylmelamine; DSPC -- distearoylphosphatidylcholine; CHOL -- cholesterol; DSPG -- distearoylphosphatidylglycerol; TRI -- triglyceride.

2/ Approximate percent precipitation of components following centrifugation at 750 G for ten minutes

3/ Concentration of HXM in mg/ml, after filtration through 5.0-micrometer and 0.45-micrometer filters.

Table 1 demonstrates that useful concentrations of HXM in aqueous solution may be achieved using the compositions of the present invention. Preferred formulations use trilaurin in the inner phase, and 100 mM glycerol dissolved in the aqueous phase. Analysis using UV/visible spectroscopy of the four trilaurin compositions tested for final HXM concentration showed no noticeable difference from the starting drug. Thin layer chromatography was also consistent with intact HXM in these cases. A repeated UV/visible spectroscopic analysis after 24 hours indicated diminished absorbance at 227 nm, suggesting a decrease in aqueous HXM from about 3.0 to 2.2 mg/ml. The later spectra were consistent with intact HXM.

An alternate procedure for formulating the present compositions, useful especially for small batches, involves dissolving each desired component in an organic solvent such as chloroform, mixing appropriate volumes of each chloroform solution, evaporating the chloroform under vacuum to obtain a lipid-drug-triglyceride film, and then adding this film to the appropriate aqueous phase as dicussed above.

EXAMPLE 2

Incorporation of Hexamethylmelamine Into Liposomal Delivery Vehicles

By way of comparison, attempts were made to incorporate HXM into the intra-bilayer phospholipid region of a liposome without use of any triglyceride. Appropriate proportions of HXM, phospholipid and cholesterol were dissolved in an organic solvent such as chloroform (distearoylphosphatidylglycerol was dissolved in 1:1 methanol:chloroform and then mixed with the chloroform solution). The solvent was then removed under reduced pressure to yield a lipid-drug film. This film was then mixed and sonicated as above in an appropriate aqueous solvent to yield small unilamellar liposomal vesicles. As above, the addition of 100 mM glycerol prevented agglomeration in some instances. Following centrifugation, the liposomes were filtered and analyzed for HXM concentrations in aqueous solution.

Results of these tests showed that addition of the anion distearoylphosphatidylglycerol increased the amount of membrane-incorporated HXM by promoting partitioning of the drug into the lipid phase. However, the final concentration of HXM achieved did not in such cases reach the desired level of at least 1.0 mg/ml. Based on these results, the desirability of the alternative aqueous solubilization vehicles disclosed herein becomes clear.

**Claims**

**Claims for the following Contracting States : AT, BE, CH, DE, FR, GB, IT, LU, NL, SE**

**1.** A composition suitable for the delivery of a substantially aqueous-insoluble active ingredient comprising vesicles in a pharmaceutically acceptable carrier, wherein said vesicles are of a size of 30 to 200 nanometers in diameter and comprise (1) a substantially aqueous-insoluble active ingredient in mixture with trilauroylglycerol and (2) an encapsulating monolayer including a phospholipid material.

**2.** The composition of claim 1 wherein the encapsulating layer includes cholesterol.

**3.** The composition of any one of the preceding claims wherein the phospholipid material is at least one phospholipid having an alkyl side chain of 12 to 20 carbon atoms in length.

**4.** The composition of claim 3 wherein the phospholipid material includes an anionic phospholipid component.

**5.** The composition of claim 1 or 2 wherein the phospholipid material comprises a mixture of dialkylphosphatidylcholine and dialkylphosphatidylglycerol compounds having alkyl side chains of 12 to 20 carbon atoms in length.

**6.** The composition of claim 5 wherein the encapsulating layer includes distearoylphosphatidylcholine, distearoylphosphatidylglycerol and cholesterol.

**7.** A composition according to any one of claims 1 to 6, wherein the active ingredient is an antitumour compound, for use in a method of treating neoplastic tumours in a human body by parenteral administration.

**8.** A composition according to claim 7 wherein the active ingredient is hexamethylmelamine.

**9.** A method of making a composition as defined in any one of the preceding claims comprising:

(1) combining said active ingredient with trilauroylglycerol, a phospholipid material and a pharmaceutically acceptable carrier;

(2) forming vesicles containing the active ingredient; and

(3) removing undesirable materials from the resulting composition.

**10.** The method of claim 9 wherein the active ingredient is hexamethylmelamine and 1 molar fraction of hexamethylmelamine is combined with about 4 molar fractions trilauroylglycerol, 2-1 molar fractions distearoylphosphatidylcholine, about 1 molar fraction cholesterol, and 0-1 molar fractions distearoylphosphatidylglycerol.

11. A method of making a composition of vesicles suitable for the delivery of a substantially aqueous-insoluble active ingredient, wherein said vesicles are of a size of 30 to 200 nanometers in diameter, comprising forming a triglyceride solution of the aqueous-insoluble active ingredient and a phospholipid and then forming vesicles comprising an outer monolayer phospholipid coat and an enclosed phase containing the active ingredient and a triglyceride material in an aqueous, pharmaceutically acceptable carrier.

**Claims for the following Contracting States : ES, GR**

1. A method of making a composition suitable for the delivery of a substantially aqueous-insoluble active ingredient comprising vesicles in a pharmaceutically acceptable carrier, wherein said vesicles are of a size of 30 to 200 nanometers in diameter and comprise (1) a substantially aqueous-insoluble ingredient in mixture with trilauroylglycerol and (2) an encapsulating monolayer including a phospholipid material, said method comprising:
   (1) combining said active ingredient with trilauroylglycerol, a phospholipid material and a pharmaceutically acceptable carrier;
   (2) forming vesicles containing the active ingredient; and
   (3) removing undesirable materials from the resulting composition.

2. The method of claim 1 wherein the encapsulating layer includes cholesterol.

3. The method of any one of the preceding claims wherein the phospholipid material is at least one phospholipid having an alkyl side chain of 12 to 20 carbon atoms in length.

4. The method of claim 3 wherein the phospholipid material includes an anionic phospholipid component.

5. The method of claim 1 or 2 wherein the phospholipid material comprises a mixture of dialkylphosphatidylcholine and dialkylphosphatidyl-glycerol compounds having alkyl side chains of 12 to 20 carbon atoms in length.

6. The method of claim 5 wherein the encapsulating layer includes distearoylphosphatidylcholine, distearoylphosphatidylglycerol and cholesterol.

7. A method according to any one of claims 1 to 6 wherein the active ingredient is an antitumour compound, for use in a method of treating neoplastic tumours in a human body by parenteral administration.

8. A method according to claim 7 wherein the active ingredient is hexamethylmelamine.

9. The method of any one of claims 1 to 8 wherein the active ingredient is hexamethylmelamine and 1 molar fraction of hexamethylmelamine is combined with about 4 molar fractions trilauroylglycerol, 2-1 molar fractions distearoylphosphatidylcholine, about 1 molar fraction cholesterol, and 0-1 molar fractions distearoylphosphatidylglycerol.

10. A method of making a composition of vesicles suitable for the delivery of a substantially aqueous-insoluble active ingredient, wherein said vesicles are of a size of 30 to 200 namometers in diameter, comprising forming a triglyceride solution of the aqueous-insoluble active ingredient and a phospholipid and then forming vesicles comprising an outer monolayer phospholipid coat and an enclosed phase containing the active ingredient and a triglyceride material in an aqueous, pharmaceutically acceptable carrier.

**Patentansprüche**
**Patentansprüche für folgende Vertragsstaaten : AT, BE, CH, DE, FR, GB, IT, LU, NL, SE**

1. Zur Verabreichung eines im wesentlichen wasserunlöslichen Wirkstoffes geeignete Zusammensetzung umfassend Vesikel in einem pharmazeutisch annehmbaren Träger, wobei die Vesikel eine Größe von 30 bis 200 nm im Durchmesser besitzen und (1) einen im wesentlichen in Wasser unlöslichen Wirkstoff in Mischung mit Trilauroylglycerin und (2) eine einkapselnde Monoschicht, die ein Phospholipidmaterial

enthält, umfassen.

2. Zusammensetzung nach Anspruch 1, dadurch gekennzeichnet, daß die einkapselnde Schicht Cholesterin enthält.

3. Zusammensetzung nach einem der vorhergehenden Ansprüche, dadurch gekennzeichnet, daß das Phospholipidmaterial mindestens ein Phospholipid mit einer Alkylseitenkette einer Länge von 12 bis 20 Kohlenstoffatomen ist.

4. Zusammensetzung nach Anspruch 3, dadurch gekennzeichnet, daß das Phospholipidmaterial eine anionische Phospholipidkomponente enthält.

5. Zusammensetzung nach Anspruch 1 oder 2, dadurch gekennzeichnet, daß das Phospholipidmaterial eine Mischung aus Dialkylphosphatidylcholin und Dialkylphosphatidylglycerinverbindungen umfaßt, die Alkylseitenketten einer Länge von 12 bis 20 Kohlenstoffatomen besitzen.

6. Zusammensetzung nach Anspruch 5, dadurch gekennzeichnet, daß die einkapselnde Schicht Distearoylphosphatidylcholin, Distearolyphosphatidylglycerin und Cholesterin enthält.

7. Zusammensetzung nach einem der Ansprüche 1 bis 6, dadurch gekennzeichnet, daß der Wirkstoff eine Antitumorverbindung zur Verwendung in einem Verfahren zur Behandlung neoplastischer Tumore im menschlichen Körper durch parenterale Verabreichung ist.

8. Zusammensetzung nach Anspruch 7, dadurch gekennzeichnet, daß der Wirkstoff Hexamethylmelamin ist.

9. Verfahren zur Herstellung einer in einem der vorhergehenden Ansprüche definierten Zusammensetzung umfassend:
   (1) Kombinieren des Wirkstoffes mit Trilauroylglycerin, einem Phospholipidmaterial und einem pharmazeutisch annehmbaren Träger;
   (2) Ausbilden von Vesikeln, die den Wirkstoff enthalten;
   (3) Entfernen unerwünschter Materialien aus der resultierenden Zusammensetzung.

10. Verfahren nach Anspruch 9, dadurch gekennzeichnet, daß der Wirkstoff Hexamethylmelamin ist und 1 Molfraktion Hexamethylmelamin mit ca. 4 Molfraktionen Trilauroylglycerin, 2-1 Molfraktionen Distearoylphosphatidylcholin, ca. 1 Molfraktion Cholesterin und 0-1 Molfraktionen Distearoylphosphatidylglycerin kombiniert wird.

11. Verfahren zur Herstellung einer Vesikelzusammensetzung, die zur Verabreichung eines im wesentlichen wasserunlöslichen Wirkstoffes geeignet ist, und worin die Vesikel eine Größe von 30 bis 200 nm im Durchmesser besitzen, dadurch gekennzeichnet, daß man eine Triglyceridlösung des wasserunlöslichen Wirkstoffes und eines Phospholipids ausbildet, und dann Vesikel ausbildet, die eine äußere Phospholipid-Monoschicht und eine darin eingeschlossene Phase, die den Wirkstoff und ein Triglyceridmaterial in einem wässerigen pharmazeutisch annehmbaren Träger enthält, umfassen.

**Patentansprüche für folgende Vertragsstaaten : ES, GR**

1. Verfahren zur Herstellung einer zur Verabreichung eines im wesentlichen wasserunlöslichen Wirkstoffes geeigneten Zusammensetzung, die Vesikel in einem pharmazeutisch annehmbaren Träger enthält, und worin die Vesikel eine Größe von 30 bis 200 nm im Durchmesser besitzen und (1) einen im wesentlichen in Wasser unlöslichen Wirkstoff in Mischung mit Trilauroylglycerin und (2) eine einkapselnde Monoschicht, die ein Phospholipidmaterial enthält, umfassen, gekennzeichnet durch die Stufen:
   (1) Kombinieren des Wirkstoffes mit Trilauroylglycerin, einem Phospholipidmaterial und einem pharmazeutisch annehmbaren Träger;
   (2) Ausbilden von Vesikeln, die den Wirkstoff enthalten; und
   (3) Entfernen unerwünschter Materialien aus der resultierenden Zusammensetzung.

2. Verfahren nach Anspruch 1, dadurch gekennzeichnet, daß die einkapselnde Schicht Cholesterin enthält.

**3.** Verfahren nach einem der vorhergehenden Ansprüche, dadurch gekennzeichnet, daß das Phospholipidmaterial mindestens ein Phospholipid mit einer Alkylseitenkette einer Länge von 12 bis 20 Kohlenstoffatomen ist.

**4.** Verfahren nach Anspruch 3, dadurch gekennzeichnet, daß das Phospholipidmaterial eine anionische Phospholipidkomponente enthält.

**5.** Verfahren nach Anspruch 1 oder 2, dadurch gekennzeichnet, daß das Phospholipidmaterial eine Mischung aus Dialkylphosphatidylcholin und Dialkylphosphatidylglycerinverbindungen umfaßt, die Alkylseitenketten einer Länge von 12 bis 20 Kohlenstoffatomen besitzen.

**6.** Verfahren nach Anspruch 5, dadurch gekennzeichnet, daß die einkapselnde Schicht Distearoylphosphatidylcholin, Distearolyphosphatidylglycerin und Cholesterin enthält.

**7.** Verfahren nach einem der Ansprüche 1 bis 6, dadurch gekennzeichnet, daß der Wirkstoff eine Antitumorverbindung zur Verwendung in einem Verfahren zur Behandlung neoplastischer Tumore im menschlichen Körper durch parenterale Verabreichung ist.

**8.** Verfahren nach Anspruch 7, dadurch gekennzeichnet, daß der Wirkstoff Hexamethylmelamin ist.

**9.** Verfahren nach einem der Ansprüche 1 bis 8, dadurch gekennzeichnet, daß der Wirkstoff Hexamethylmelamin ist und 1 Molfraktion Hexamethylmelamin mit ca. 4 Molfraktionen Trilauroylglycerin, 2-1 Molfraktionen Distearoylphosphatidylcholin, ca. 1 Molfraktion Cholesterin und 0-1 Molfraktionen Distearoylphosphatidylglycerin kombiniert wird.

**10.** Verfahren zur Herstellung einer Vesikelzusammensetzung, die zur Verabreichung eines im wesentlichen wasserunlöslichen Wirkstoffes geeignet ist, und worin die Vesikel eine Größe von 30 bis 200 nm im Durchmesser besitzen, dadurch gekennzeichnet, daß man eine Triglyceridlösung des wasserunlöslichen Wirkstoffes und eines Phospholipids ausbildet, und dann Vesikel ausbildet, die eine äußere Phospholipid-Monoschicht und eine darin eingeschlossene Phase, die den Wirkstoff und ein Triglyceridmaterial in einem wässerigen pharmazeutisch annehmbaren Träger enthält, umfassen.

**Revendications**
**Revendications pour les Etats contractants suivants : AT, BE, CH, DE, FR, GB, IT, LU, NL, SE**

**1.** Composition convenant à la délivrance d'un ingrédient actif essentiellement insoluble dans l'eau, comprenant des vésicules dans un véhicule pharmaceutiquement acceptable, dans laquelle lesdites vésicules ont un diamètre de 30 à 200 nanomètres et comprennent (1) un ingrédient actif essentiellement insoluble dans l'eau en mélange avec du trilauroylglycérol et (2) une monocouche d'encapsulation comprenant une matière phospholipidique.

**2.** Composition selon la revendication 1, dans laquelle la couche d'encapsulation comprend du cholestérol.

**3.** Composition selon l'une quelconque des revendications précédentes, dans laquelle la matière phospholipidique est au moins un phospholipide ayant une chaîne latérale alkyle longue de 12 à 20 atomes de carbone.

**4.** Composition selon la revendication 3, dans laquelle la matière phospholipidique comprend un composant phospholipidique anionique.

**5.** Composition selon la revendication 1 ou 2, dans laquelle la matière phospholipidique comprend un mélange de composés dialkylphosphatidylcholine et dialkylphosphatidylglycérol ayant des chaînes latérales alkyles longues de 12 à 20 atomes de carbone.

**6.** Composition selon la revendication 5, dans laquelle la couche d'encapsulation comprend de la distéaroylphosphatidylcholine, du distéaroylphosphatidylglycérol et du cholestérol.

**7.** Composition selon l'une quelconque des revendications 1 à 6, dans laquelle l'ingrédient actif est un composé antitumoral, pour l'utilisation dans un procédé de traitement des tumeurs néoplasiques de l'organisme humain par administration parentérale.

**8.** Composition selon la revendication 7, dans laquelle l'ingrédient actif est l'hexaméthylmélamine.

**9.** Procédé de préparation d'une composition comme définie dans l'une quelconque des revendications précédentes comprenant les étapes consistant
(1) à combiner **ledit** ingrédient actif avec du trilauroylglycérol, une matière phospholipidique et un véhicule pharmaceutiquement acceptable ;
(2) à former des vésicules contenant l'ingrédient actif ; et
(3) à éliminer des matières indésirables de la composition obtenue.

**10.** Procédé selon la revendication 9, dans lequel l'ingrédient actif est l'hexaméthylmélamine et 1 fraction molaire d'hexaméthylmélamine est combinée avec environ 4 fractions molaires de trilauroylglycérol, 2 à 1 fractions molaires de distéaroylphosphatidylcholine, environ 1 fraction molaire de cholestérol et 0 à 1 fraction molaire de distéaroylphosphatidylglycérol.

**11.** Procédé de préparation d'une composition de vésicules convenant à la délivrance ingrédient actif essentiellement insoluble dans l'eau, dans laquelle lesdites vésicules ont un diamètre de 30 à 200 nm, comprenant les étapes consistant à former une solution triglycéridique de l'ingrédient actif insoluble dans l'eau et d'un phospholipide, puis à former les vésicules comprenant un revêtement extérieure contenant l'ingrédient actif et une matière triglycéridique, dans un véhicule aqueux pharmaceutiquement acceptable.

**Revendications pour les Etats contractants suivants : ES, GR**

**1.** Procédé de préparation d'une composition convenant à la délivrance d'un ingrédient actif essentiellement insoluble dans l'eau, comprenant des vésicules dans un véhicule pharmaceutiquement acceptable, dans laquelle lesdites vésicules ont un diamètre de 30 à 200 nanomètres et comprennent (1) un ingrédient actif essentiellement insoluble dans l'eau en mélange avec du trilauroylglycérol et (2) une monocouche d'encapsulation comprenant une matière phospholipidique, ledit procédé comprenant les étapes consistant :
(1) à combiner **ledit** ingrédient actif avec du trilauroylglycérol, une matière phospholipidique et un véhicule pharmaceutiquement acceptable ;
(2) à former des vésicules contenant l'ingrédient actif ; et
(3) à éliminer des matières indésirables de la composition obtenue.

**2.** Procédé selon la revendication 1, dans lequel la couche d'encapsulation comprend du cholestérol.

**3.** Procédé selon l'une quelconque des revendications précédentes, dans lequel la matière phospholipidique est au moins un phospholipide ayant une chaîne latérale alkyle longue de 12 à 20 atomes de carbone.

**4.** Procédé selon la revendication 3, dans lequel la matière phospholipidique comprend un composant phospholipidique anionique.

**5.** Procédé selon la revendication 1 ou 2, dans lequel la matière phospholipidique comprend un mélange de composés dialkylphosphatidylcholine et dialkylphosphatidylglycérol ayant des chaînes latérales alkyles longues de 12 à 20 atomes de carbone.

**6.** Procédé selon la revendication 5, dans lequel la couche d'encapsulation comprend de la distéaroylphosphatidylcholine, du distéaroylphosphatidylglycérol et du cholestérol.

**7.** Procédé selon l'une quelconque des revendications 1 à 6, dans lequel l'ingrédient actif est un composé antitumoral, pour l'utilisation dans un procédé de traitement des tumeurs néoplasiques de l'organisme humain par administration parentérale.

8. Procédé selon la revendication 7, dans lequel l'ingrédient actif est l'hexaméthylmélamine.

9. Procédé selon l'une quelconque des revendications 1 à 8, dans lequel l'ingrédient actif est l'hexaméthylmélamine et 1 fraction molaire d'hexaméthylmélamine est combinée avec environ 4 fractions molaires de trilauroylglycérol, 2 à 1 fractions molaires de distéaroylphosphatidylcholine, environ 1 fraction molaire de cholestérol et 0 à 1 fraction molaire de distéaroylphosphatidylglycérol.

10. Procédé de préparation d'une composition de vésicules convenant à la délivrance ingrédient actif essentiellement insoluble dans l'eau, dans laquelle lesdites vésicules ont un diamètre de 30 à 200 nm, comprenant les étapes consistant à former une solution triglycéridique de l'ingrédient actif insoluble dans l'eau et d'un phospholipide, puis à former des vésicules comprenant un revêtement extérieur fait d'une monocouche phospholipidique et d'une phase intérieure contenant l'ingrédient actif et une matière triglycéridique, dans un véhicule aqueux pharmaceutiquement acceptable.

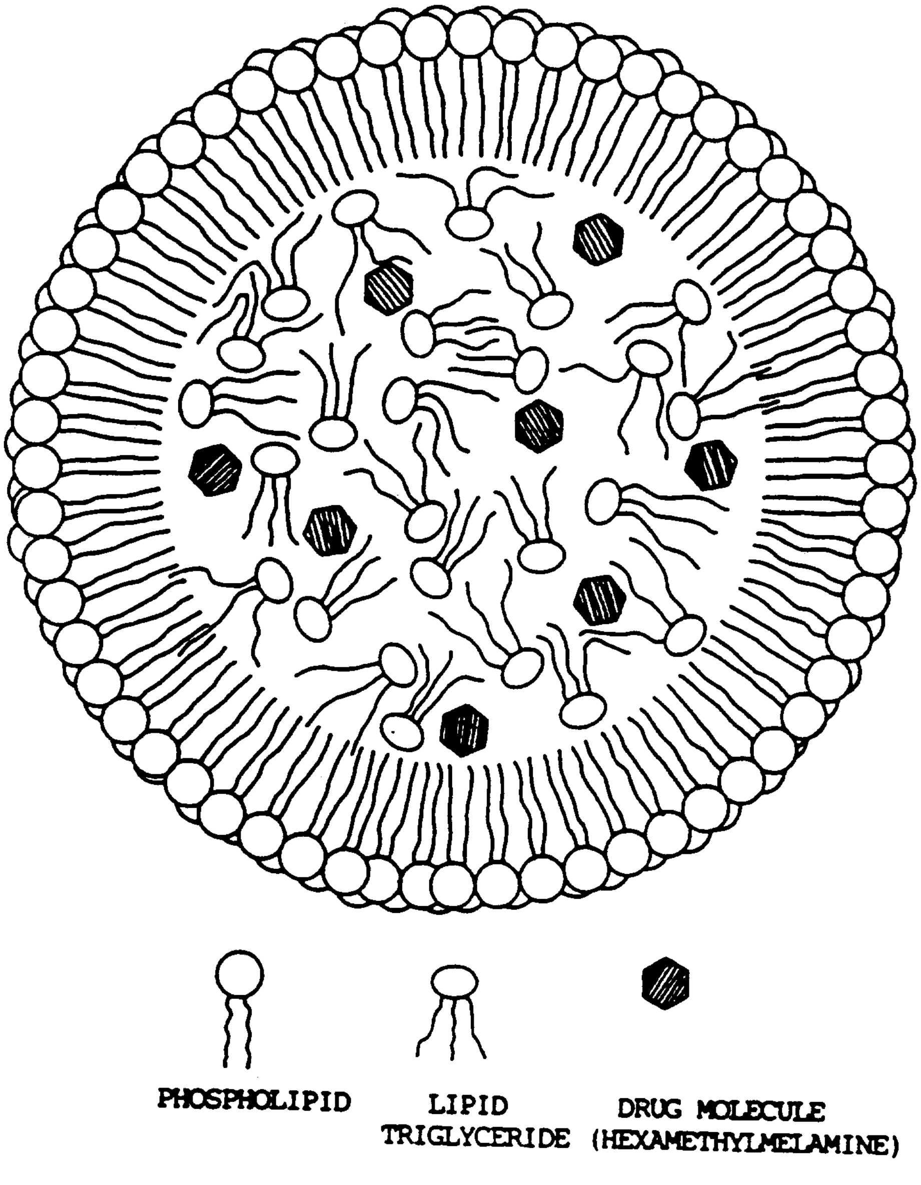
PHOSPHOLIPID
LIPID
TRIGLYCERIDE
DRUG MOLECULE
(HEXAMETHYLMELAMINE)